# EUROPEAN PATENT APPLICATION

(11) **EP 4 382 541 A1**
(43) Date of publication of application: **12.06.2024**
(21) Application number: 22909337.2
(22) Date of filing: 24.08.2022
(51) Int. Cl.: C07K 16/46, C07K 16/30, A61K 39/395, C12N 15/13

(54) **BISPECIFIC ANTIBODY AND USE THEREOF**

(30) Priority: 20.12.2021 CN 202111564703
(71) Applicant: Shanghai Nk Cell Tech Co., Ltd., Shanghai 201318 (CN)
(72) Inventor: XIAO, Weihua, Shanghai 201318 (CN); TIAN, Zhigang, Shanghai 201318 (CN); LI, Yangyang, Shanghai 201318 (CN); XIE, Siqi, Shanghai 201318 (CN); CHEN, Minhua, Shanghai 201318 (CN)
(74) Representative: DehnsGermany Partnerschaft von Patentanwälten
(86) International application number: PCT/CN2022/114434
(87) International publication number: WO 2023/116010

(57) **Abstract**

Provided are a bispecific antibody and a use thereof. The bispecific antibody includes an IL-21 molecule and a CD16a antibody. The CD16a antibody includes a CD16a single-chain antibody and FC. The N-terminus of the FC is linked to the C-terminus of the single-chain antibody. The CD16a single-chain antibody includes a heavy chain variable region and a light chain variable region. The C-terminus of the FC is linked to the N-terminus of the IL21 molecule. The prepared bispecific antibody can simultaneously target CD16 and IL-21 receptors and can also activate NK cells. It has a long half-life period, exhibiting stronger anti-tumor and antiviral capabilities than single-target antibodies. It has the application potential of promoting NK cell proliferation and can be used for in-vitro culture and expansion of NK cells, and the expanded NK cells have good cytotoxicity.

## Description

### PRIORITY INFORMATION

This application claims priority to and the benefit of Patent Application No. 202111564703.3, filed on December 20, 2021 to the China National Intellectual Property Administration, the entirety of which is incorporated herein by reference.

### FIELD

The present disclosure belongs to the field of biomedicines, particularly to bispecific antibodies and use thereof, and more particularly to recombinant antibodies, immune cells, nucleic acid molecules, expression vectors, recombinant cells, compositions, uses of the above for the preparation of a medicament, and a kit.

### BACKGROUND

Bispecific antibodies are antibodies that specifically bind to two antigenic sites simultaneously. NK cells are the main effector cells of the innate immune system. Compared with T cells, NK cells can kill tumor cells and virus-infected cells without MHC restriction by using perforin and granzymes as well as the related mechanisms without prior stimulation. NK cells express a large number of activating receptors, which can effectively recognize the stress ligands produced by tumor cells or infected cells, thereby effectively killing tumor cells. However, at the same time, tumor cells also secrete many immunosuppressive molecules to produce an immunosuppressive tumor microenvironment, such that the activity of NK cells in this environment and the exertion of anti-tumor ability are inhibited, thereby allowing NK cells to be in a state of exhaustion. Currently, there are also several strategies for restoring NK cell exhaustion in the tumor microenvironment, for example, immune checkpoint blocking, activating antibodies, activating cytokines, etc., which can provide activation signals to NK cells or block inhibitory signals in the tumor microenvironment, thereby restoring the normal activation state of NK cells and exerting anti-tumor or antiviral functions.

CD16a is an important class of activating receptors expressed in NK cells. When CD16a is activated by its ligand or antibody binding, it induces a variety of cascade activation reactions, releasing granzyme, perforin, inflammatory cytokines, and chemokines, which brings a strong activation effect and enhancement of anti-tumor ability for NK cells. IL-21 receptor (IL-21R) is a constitutively expressed cytokine receptor in NK cells. For NK cells in mice, the activation of IL-21R through the recognition and binding of its corresponding ligand IL-21 molecule may promote more granules of these NK cells, up-regulate the expression of perforin, increase the secretion of IFN-γ, and promote the effector activity; and for human NK cells, the activation of IL-21R can enhance IFN-γ secretion, increase proliferation and NK cytotoxic activity, while accelerating NK cell maturation and NK cell receptor expression. Therefore, the development of highly targeted bispecific antibodies is of great value to the prevention and treatment of tumors.

### SUMMARY

The present disclosure is based on the Applicant's discovery and recognition of the following facts and problems:
In the present disclosure, the Applicant has designed a recombinant bispecific antibody by combining the CD16a antibody and the IL-21 molecule. The bispecific recombinant antibody can specifically bind to CD16a and IL-21 receptor (IL-21R), thereby bringing the IL-21 molecule and the CD16a antibody to the vicinity of NK cells to bind to CD16a and/or IL-21R on the NK cells. In this way, various cascade activation reactions can be induced, activating the NK cells and restoring the exhaustion of NK cells in the tumor microenvironment. At the same time, IFN-γ secretion can be intensified, proliferation and NK cytotoxic activity can be enhanced, while NK cell maturation and NK cell receptor expression can be accelerated, thereby killing tumor cells or virus-infected cells. The recombinant bispecific antibody has a relatively high CD16a and IL-21R binding activity and a relatively high anti-tumor and antiviral ability.

Thus, in a first aspect, the present disclosure provides a recombinant antibody. According to an embodiment of the present disclosure, the recombinant antibody includes IL-21 molecules and a CD 16a antibody. The CD 16a antibody includes a CD16a single-chain antibody and an Fc region. The N-terminus of the Fc region is linked to the C-terminus of the single-chain antibody. The CD16a single-chain antibody includes a heavy chain variable region, a light chain variable region. The C-terminus of the FC is linked to the N-terminus of the IL21 molecule. The recombinant antibody according to the embodiments of the present disclosure can effectively bind to CD16a and IL-21R, it has strong *in vivo* and *in vitro* binding activity and can effectively activate NK cells, and it also has a long *in vivo* half-life and significant anti-tumor and antiviral effects.

In a second aspect, the present disclosure provides a nucleic acid molecule. According to an embodiment of the present disclosure, the nucleic acid molecule encodes the recombinant antibody of the first aspect. The recombinant antibody encoded by the nucleic acid molecule according to the embodiments of the present disclosure can effectively bind to CD16a and IL-21R, it has strong *in vivo* and *in vitro* binding activity and can effectively activate NK cells, and it also has a long *in vivo* half-life and significant anti-tumor and antiviral effects.

In a third aspect, the present disclosure provides an expression vector. According to an embodiment of the present disclosure, the expression vector carries the nucleic acid molecule of the second aspect. The expression vector may include optional control sequences operably linked to the nucleic acid molecule. The control sequences are one or more control sequences that direct expression of the nucleic acid molecule in a host. The expression vector provided in the embodiments of the present disclosure can efficiently express the recombinant antibodies in suitable host cells, so as to effectively activate NK cells for treating or preventing diseases caused by tumor or virus infection.

In a fourth aspect, the present disclosure provides a recombinant cell. According to an embodiment of the present disclosure, the recombinant cell carries the nucleic acid molecule of the second aspect, the expression vector of the third aspect, or the recombinant antibody of the first aspect. The recombinant cell is obtained by transfecting or transforming the expression vector. According to an embodiment of the present disclosure, the recombinant cell can highly express the above recombinant antibody under suitable conditions, and the recombinant cell can be effectively used for treating or preventing diseases caused by tumor or virus infection and can effectively activate NK cells.

In a fifth aspect, the present disclosure provides a composition. According to an embodiment of the present disclosure, the composition includes the recombinant antibody according to the first aspect, the nucleic acid molecule according to the second aspect, the expression vector according to the third aspect, or the recombinant cell according to the fourth aspect. As described above, the recombinant antibody according to the embodiments of the present disclosure can effectively bind to CD16a and IL-21R protein molecules, so as to specifically recognize the NK cells highly expressing the IL-21R and/or CD16a. Thus, the NK cells can be effectively activated, and the composition including the recombinant antibody also have significant effects in treating or preventing diseases caused by tumor or virus infection, having significant anti-tumor and antiviral capabilities.

In a sixth aspect of the present disclosure, the use of a recombinant antibody according to the first aspect, a nucleic acid molecule according to the second aspect, an expression vector according to the third aspect, a recombinant cell according to the fourth aspect or a composition according to the fifth aspect for the preparation of a medicament is provided. According to an embodiment of the present disclosure, the medicament is for treating or preventing tumor or virus infection. As described above, the recombinant antibodies of the embodiments of the present disclosure can effectively bind to CD16a and IL-21R proteins, so as to specifically recognize NK cells highly expressing the IL-21R and/or CD16a, and the medicament prepared using the recombinant antibodies and the corresponding series of substances also have significant effects on the treatment or prevention of tumor or virus infection, with significant anti-tumor and antiviral capabilities.

In a seventh aspect of the present disclosure, there is provided the use of a recombinant antibody according to the first aspect, a nucleic acid molecule according to the second aspect, an expression vector according to the third aspect, and a recombinant cell according to the fourth aspect in culture and expansion of NK cells. Recombinant antibodies, nucleic acid molecules, expression vectors, and recombinant cells according to embodiments of the present disclosure can be used to promote proliferation, and expansion of the NK cells *in vitro,* and the expanded NK cells have better cytotoxicity.

In an eighth aspect, the present disclosure provides a medicament. According to an embodiment of the present disclosure, the medicament includes the recombinant antibody of the first aspect, the nucleic acid molecule of the second aspect, the expression vector of the third aspect, the recombinant cell of the fourth aspect, or the composition of the fifth aspect, and the medicament is used for treating or preventing tumor or virus infection. As described above, the recombinant antibody according to the embodiments of the present disclosure can effectively bind to CD 16a and IL-21R proteins, so as to specifically recognize NK cells highly expressing the IL-21R and/or CD16a, and the medicament prepared using the recombinant antibodies and the corresponding series of substances also has significant effects in treating or preventing tumor or virus infection, having significant anti-tumor and antiviral capabilities.

In a ninth aspect, the present disclosure provides a kit. According to an embodiment of the present disclosure, the recombinant antibody of the first aspect is comprised. The recombinant antibody according to the embodiments of the present disclosure can efficiently bind to CD16a and IL-21R protein molecules, thereby specifically recognizing NK cells specifically expressing high levels of the IL-21R or CD 16a. Thus, the recombinant antibodies can be used to prepare kits for detecting CD16a and/or IL-21R, which can be used in scientific studies, such as qualitative or quantitative detection of CD16a and/or IL-21R protein molecules in biological samples, to obtain satisfactory biological samples, etc.

In a tenth aspect, the present disclosure provides uses of the recombinant antibody, nucleic acid molecule, expression vector, recombinant cell, composition, or medicament as described above in the treatment or prevention of tumor or virus infection. As described above, the recombinant antibody can specifically recognize the above-mentioned CD16a and IL-21R proteins, and further can specifically recognize NK cells specifically highly expressing the IL-21R or CD16a. Thus, NK cells can be effectively activated, IFN-γ secretion can be enhanced, proliferation and NK cytotoxic activity can be enhanced, tumor cells can be effectively killed, NK cell maturation and NK cell receptor expression can be simultaneously accelerated, and exhaustion of NK cells in the tumor microenvironment can be effectively restored. Therefore, the recombinant antibody, composition including the recombinant antibody, medicament according to the embodiments of the present disclosure, or a series of substances capable of expressing the recombinant antibody under appropriate conditions such as the above-mentioned nucleic acid molecule, expression vector, recombinant cell, etc. can effectively activate NK cells to treat or prevent tumor or virus infection.

In an eleventh aspect, the present disclosure provides a method for treating or preventing tumor or virus infection. According to some specific embodiments of the present disclosure, the method include: administering to the subject at least one of 1) the above-mentioned recombinant antibodies; 2) the above-mentioned nucleic acid molecule; 3) the above-mentioned expression vector; 4) the above-mentioned recombinant cell; 5) the above-mentioned composition; or 6) the above-mentioned medicament. As described above, the recombinant antibody can specifically recognize the above-mentioned CD16a and IL-21R proteins, and it further can specifically recognize NK cells specifically highly expressing the IL-21R or CD16a. Thus, NK cells can be effectively activated, IFN-γ secretion can be enhanced, proliferation and NK cytotoxic activity can be enhanced, tumor cells can be effectively killed, NK cell maturation and NK cell receptor expression can be simultaneously accelerated, and exhaustion of NK cells in the tumor microenvironment can be effectively restored. Therefore, the recombinant antibody, the composition including the recombinant antibody, medicament according to the embodiments of the present disclosure, or a series of substances capable of expressing the recombinant antibody under appropriate conditions such as the above-mentioned nucleic acid molecule, expression vector, recombinant cell, etc. can effectively activate NK cells and can thus treat or prevent tumor or virus infection.

Additional aspects and advantages of the present disclosure will be illustrated in part in the description which follows and, in part, will be obvious from the description, or may be learned by practice of the present disclosure.

### BRIEF DESCRIPTION OF THE DRAWINGS

The foregoing and/or additional aspects and advantages of the present disclosure will become apparent and readily appreciated from the following description of the embodiments in conjunction with the accompanying drawings of which:

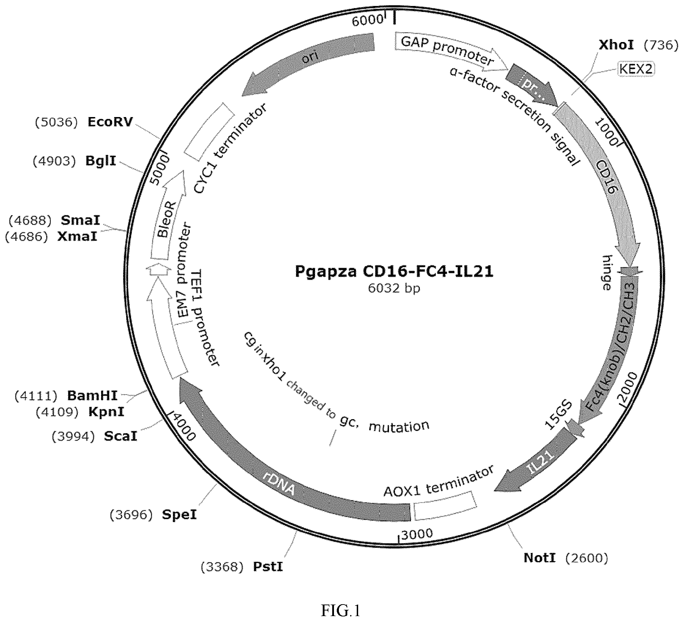

FIG. 1 is a schematic diagram of a bispecific antibody expression vector according to an embodiment of the present disclosure.

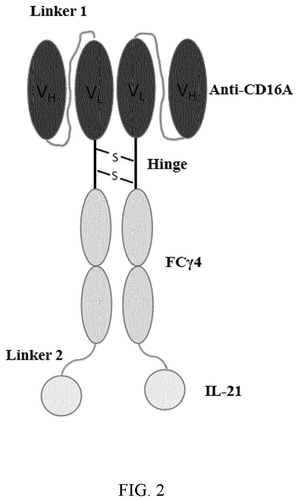

FIG. 2 is a schematic structural diagram of a recombinant bispecific antibody according to an embodiment of the present disclosure, in which Linker 1 represents connecting peptide 1, Linker 2 represents connecting peptide 2, Anti-CD16A represents a CD16A single-chain antibody, and Hinge represents a hinge region;

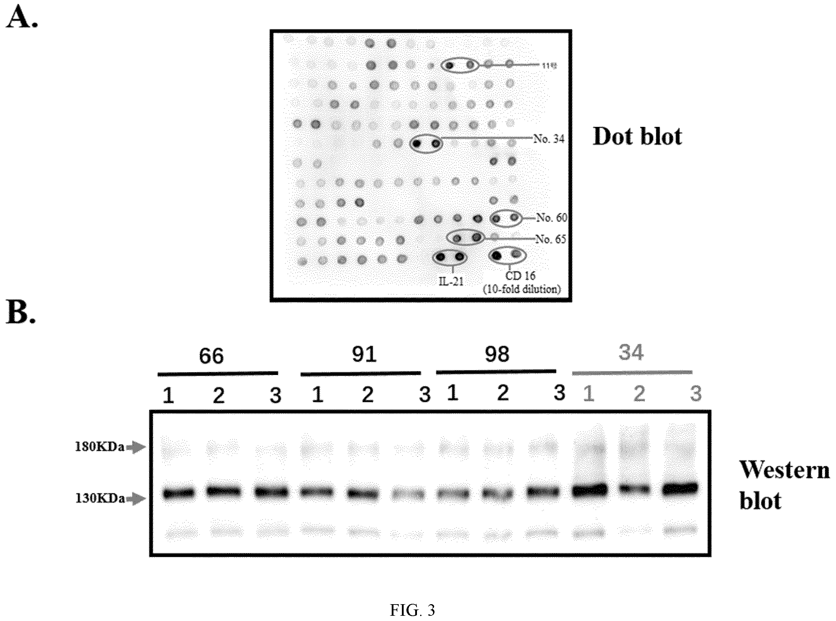

FIG. 3 illustrates graphs showing the analysis results of recombinant bispecific antibody anti-CD16A-FCγ4-IL21 expression clone screening according to an embodiment of the present disclosure, in which: FIG. 3-A is DB, Dot blot and Dot hybridization detection results; and FIG. 3-B is detection results of Western blot (WB), immunoblotting (anti-human FCγ4 antibody).

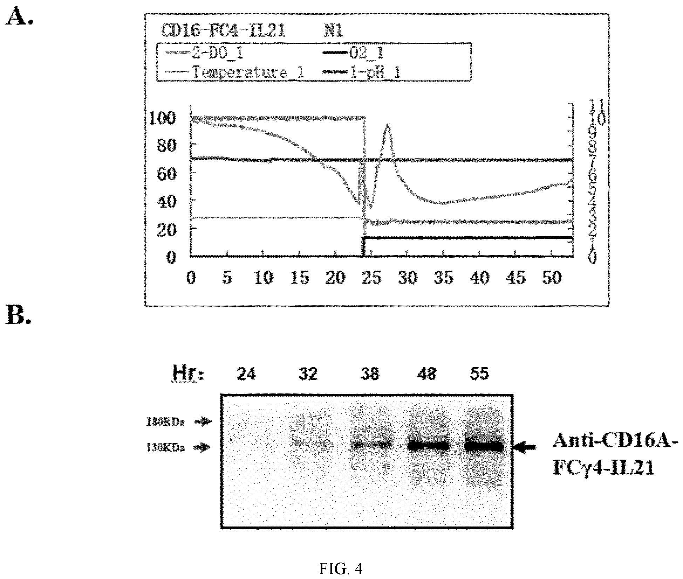

FIG. 4 illustrates graphs showing the analysis results of fermentation expression of recombinant bispecific antibody anti-CD16A-FCγ4-IL21 according to an embodiment of the present disclosure, in which: FIG. 4-A is a graph showing the results of monitoring various parameters of the fermentation process; and FIG. 4-B is a graph showing the detection results of target protein accumulation time points.

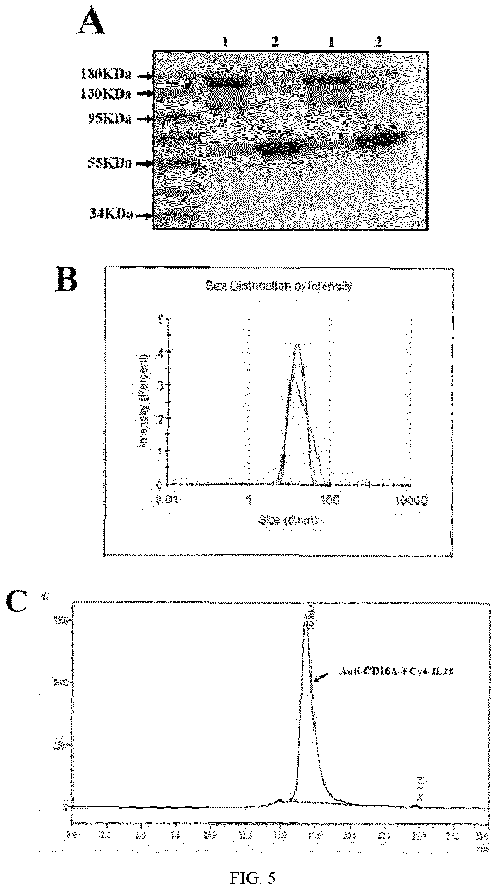

FIG. 5 illustrates graphs showing the results of purification and physicochemical identification of recombinant bispecific antibody anti-CD 16A-FCγ4-IL21 according to an embodiment of the present disclosure, in which: FIG. 5-A is a graph showing the results of the SDS-PAGE assay for protein purity of anti-CD16A-FCγ4-IL21; FIG. 5-B is a graph showing the results of dynamic light scattering assay for anti-CD 16A-FCγ4-IL21, three solid lines representing triplicates of the same treatment; and FIG. 5-C is a graph showing the results of HPLC detection of anti-CD 16A-FCγ4-IL21.

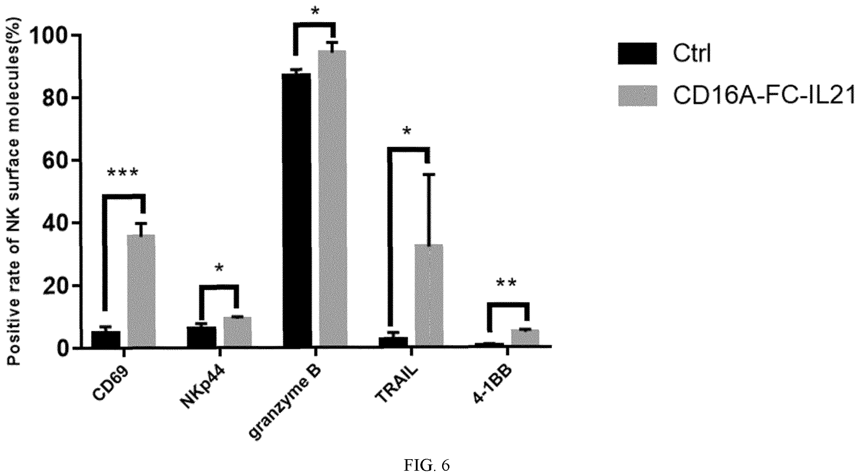

FIG. 6 illustrates graphs showing the activity detection results of NK cells activated by recombinant bispecific antibody anti-CD16A-FCγ4-IL21 *in vitro* according to an embodiment of the present disclosure, in which the abscissa represents a molecule whose expression on the surface of NK cells is changed in PBMC after the activation of anti-CD16A-FCγ4-IL21, and the ordinate, Positive rate of NK surface molecules (%), represents the positive rate (expression) of the molecule on the surface of NK cells.

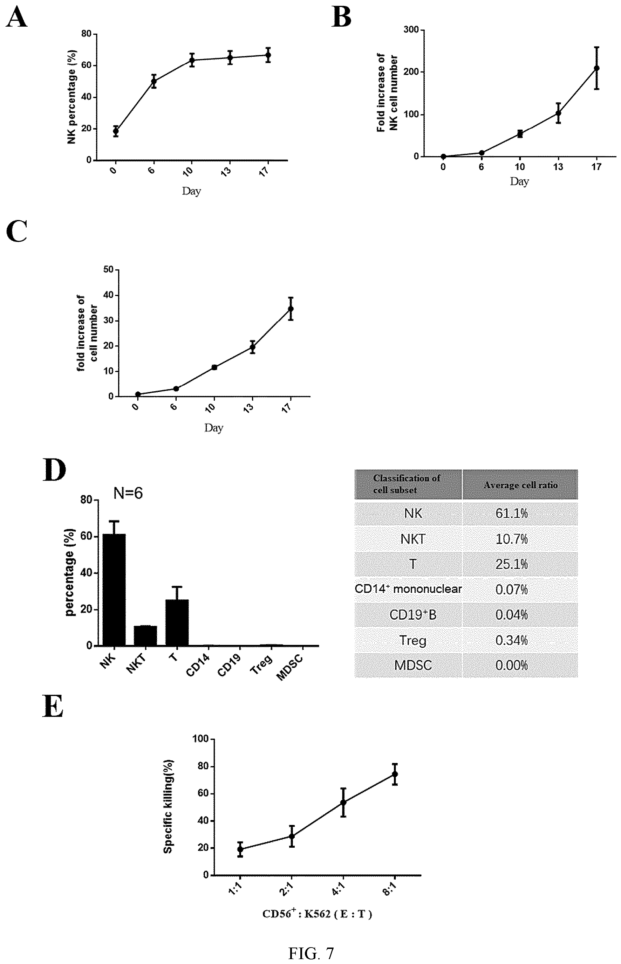

FIG. 7 illustrates graphs showing the detection results of NK cell expansion induced *in vitro* by the bispecific antibody molecule anti-CD16a-FCγ4-IL21 and the expansion effect according to an embodiment of the present disclosure, in which: FIG. 7-A shows the NK cell expansion induced by the bispecific antibody molecule anti-CD16a-FCγ4-IL21 *in vitro,* where the abscissa Day represents the number of days, and the ordinate NK Percentage (%) represents the proportion of NK cells; FIG. 7-B is a graph showing the results of NK cell expansion induced by the bispecific antibody molecule anti-CD 16a-FCγ4-IL21 *in vitro,* where the abscissa Day represents the number of days, and the ordinate Fold increase of NK cell number represents the expansion factor of NK cells in the expansion system; FIG. 7-C is a graph showing the results of NK cell expansion induced by the bispecific antibody molecule anti-CD16a-FCγ4-IL21 *in vitro,* where the abscissa Day represents the number of days and the ordinate Fold increase of cell number represents the expansion factor of total cells in the expansion system; FIG. 7-D is a graph showing the results of cell subtype analysis obtained after *in vitro* expansion of PBMC-derived NK cells with the bispecific antibody molecule anti-CD16a-FCγ4-IL21, where the abscissa represents the cell subset classification and the ordinate Percentage (%) represents the average cell proportion; and FIG. 7-E is a graph showing the results of the cytotoxicity assay ofNK cells expanded by the bispecific antibody molecule anti-CD 16a-FCγ4-IL21, where the abscissa represents the ratio of CD56 positive cells to K562 cells and the ordinate Specific killing (%) represents the ratio of killing specific cells.

### DETAILED DESCRIPTION

Reference will now be made in detail to the embodiments of the present disclosure, examples of which are illustrated in the accompanying drawings. The embodiments described below with reference to the figures are exemplary and are intended to be illustrative of the present disclosure and are not to be construed as limiting the present disclosure.

Furthermore, the terms "first" and "second" are used for descriptive purposes only and are not to be construed as indicating or implying relative importance or implicitly indicating the number of technical features indicated. Thus, a feature defined as "first" or "second" may explicitly or implicitly include at least one such feature. In the description of the present disclosure, the meaning of "plurality" is at least two, e.g. two, three, etc. unless specifically and specifically limited otherwise.

### Recombinant antibody

In one aspect, the present disclosure provides a recombinant antibody including an IL-21 molecule and a CD16a antibody. The CD16a antibody includes a CD16a single-chain antibody and an Fc region. The N-terminus of the Fc region is linked to the C-terminus of the single-chain antibody. The CD16a single-chain antibody includes a heavy chain variable region, a light chain variable region. The C-terminus of the FC is linked to the N-terminus of the IL21 molecule. The recombinant antibody according to the embodiments of the present disclosure can effectively bind to CD16a and IL-21R, it has strong *in vivo* and *in vitro* binding activity and can effectively activate NK cells, and it also has a long *in vivo* half-life and significant anti-tumor and antiviral effects.

According to some specific embodiments of the present disclosure, the above-mentioned recombinant antibody may further include at least one of the following additional technical features.

According to some specific embodiments of the present disclosure, the recombinant antibody further includes a connecting peptide 1. The N-terminus of the connecting peptide 1 is linked to the C-terminus of the heavy chain variable region of the CD16a single-chain antibody, and the C-terminus of the connecting peptide 1 is linked to the N-terminus of the light chain variable region of the CD16a single-chain antibody.

According to some specific embodiments of the present disclosure, the connecting peptide 1 has an amino acid sequence as set forth in SEQ ID NO: 1.
GGGGSGGGGSGGGGS (SEQ ID NO: 1).

According to some specific embodiments of the present disclosure, the recombinant antibody further includes a connecting peptide 2. The N-terminus of the connecting peptide 2 is linked to the C-terminus of the IL-21 molecule, and the C-terminus of the connecting peptide 2 is linked to the N-terminus of the Fc region.

According to some specific embodiments of the present disclosure, the connecting peptide 2 has an amino acid sequence as set forth in SEQ ID NO: 2.
GGGGSGGGGSGGGGS (SEQ ID NO: 2).

According to some specific embodiments of the present disclosure, at least a portion of the Fc region is derived from at least one of a murine antibody, a human antibody, a primate antibody, or a mutant thereof.

According to some specific embodiments of the present disclosure, at least a portion of the Fc region is derived from human IgG or a mutant thereof.

According to some specific embodiments of the present disclosure, at least a portion of the Fc region is derived from Fcγ4 or a mutant thereof.

According to some specific embodiments of the present disclosure, at least a portion of the Fc region is derived from human Fcγ4 or a mutant thereof.

According to some specific embodiments of the present disclosure, the Fc region has an amino acid sequence as set forth in SEQ ID NO: 3.

APEFEGGPSVFLFPPKPKDTLMISRTPEVTCVVVDVSQEDPEVQFNWYVDG
VEVHNAKTKPREEQFQSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGK (SEQ ID
NO: 3).

According to some specific embodiments of the present disclosure, the recombinant antibody has an amino acid sequence as set forth in SEQ ID NO: 4.

QVQLVQSGAEVKKPGESLKVSCKASGYTFTSYYMHWVRQAPGQGLEWM
GIINPSGGSTSYAQKFQGRVTMTRDTSTSTVYMELSSLRSEDTAVYYCARGSAYYYDF
ADYWGQGTLVTVSSGGGGSGGGGSGGGGSSYVLTQPSSVSVAPGQTATISCGGHNIG
SKNVHWYQQRPGQSPVLVIYQDNKRPSGIPERFSGSNSGNTATLTISGTQAMDEADYY
CQVWDNYSVLFGGGTKLTVLESKYGPPCPPCPAPEFEGGPSVFLFPPKPKDTLMISRTP
EVTCVVVDVSQEDPEVQFNWYVDGVEVHNAKTKPREEQFQSTYRVVSVLTVLHQD
WLNGKEYKCKVSNKGLPSSIEKTISKAKGQPREPQVYTLPPSQEEMTKNQVSLTCLVK
GFYPSDIAVEWESNGQPENNYKTTPPVLDSDGSFFLYSRLTVDKSRWQEGNVFSCSV
MHEALHNHYTQKSLSLSLGKGGGGSGGGGSGGGGSQDRHMIRMRQLIDIVDQLKNY
VNDLVPEFLPAPEDVETNCEWSAFSCFQKAQLKSANTGNNERIINVSIKKLKRKPPSTN
AGRRQKHRLTCPSCDSYEKKPPKEFLERFKSLLQKMIHQHLSSRTHGSEDS (SEQ ID
NO: 4).

### Preventive or therapeutic composition

In an aspect, the present disclosure provides a nucleic acid molecule encoding the recombinant antibody as described above. The recombinant antibody encoded by the nucleic acid molecule according to the embodiments of the present disclosure can effectively bind to CD16a and IL-21R, it has strong *in vivo* and *in vitro* binding activity and can effectively activate NK cells, and it also has a long *in vivo* half-life and significant anti-tumor and antiviral effects.

According to some specific embodiments of the present disclosure, the nucleic acid molecule has a nucleotide sequence as set forth in SEQ ID NO: 5.

CAAGTTCAATTGGTTCAATCTGGTGCTGAGGTTAAGAAACCAGGTGAGT
CTTTGAAGGTTTCTTGTAAGGCTTCTGGTTATACTTTCACTTCTTACTATATGCATTG
GGTTAGACAAGCTCCAGGTCAAGGTTTGGAGTGGATGGGTATTATTAATCCATCTG
GTGGTTCTACTTCTTATGCTCAAAAGTTCCAAGGTAGAGTTACTATGACTAGAGATA
CTTCTACTTCTACTGTTTATATGGAGTTGTCTTCTTTGAGATCTGAAGATACTGCTGT
TTATTATTGTGCTAGAGGTTCTGCTTATTATTATGATTTCGCTGATTATTGGGGTCAA
GGTACTTTGGTTACTGTCTCTTCTGGTGGTGGTTCTGGTGGTGGTTCTGG
TGGTGGTGGTTCCTCTTACGTCTTGACTCAACCATCTTCTGTCTCTGTTGCTCCAGG
TCAAACTGCTACTATTTCTTGTGGTGGTCATAATATTGGTTCTAAGAATGTTCATTGG
TATCAACAAAGACCAGGTCAATCTCCAGTCTTGGTCATCTATCAAGATAACAAGAG
ACCATCTGGTATTCCAGAGAGATTCTCTGGTTCTAATTCTGGTAATACTGCTACTTT
GACTATTTCTGGTACTCAAGCTATGGATGAAGCTGATTACTATTGTCAAGTTTGGGA
TAATTATTCTGTCTTGTTCGGTGGTGGTACTAAGTTGACTGTTTTGGAGTCCAAATA
TGGTCCCCCATGCCCACCATGCCCAGCACCTGAGTTCGAAGGGGGACCATCAGTC
TTCCTGTTCCCCCCAAAACCCAAGGACACTCTCATGATCTCCCGGACCCCTGAGGT
CACGTGCGTGGTGGTGGACGTGAGCCAGGAAGACCCCGAGGTCCAGTTCAACTG
GTACGTGGATGGCGTGGAGGTGCATAATGCCAAGACAAAGCCGCGGGAGGAGCA
GTTCCAAAGCACGTACCGTGTGGTCAGCGTCCTCACCGTCCTGCACCAGGACTGG
CTGAACGGCAAGGAGTACAAGTGCAAGGTCTCCAACAAAGGCCTCCCGTCCTCCA
TCGAGAAAACCATCTCCAAAGCCAAAGGGCAGCCCCGAGAGCCACAGGTGTACA
CCCTGCCCCCATCCCAGGAGGAGATGACCAAGAACCAGGTCAGCCTGacCTGCCTG
GTCAAAGGCTTCTACCCCAGCGACATCGCCGTGGAGTGGGAGAGCAATGGGCAGC
CGGAGAACAACTACAAGACCACGCCTCCCGTGCTGGACTCCGACGGCTCCTTCTT
CCTCTACAGCAGACTAACCGTGGACAAGAGCAGGTGGCAGGAGGGGAATGTCTTC
TCATGCTCCGTGATGCATGAGGCTCTGCACAACCACTACACACAGAAGAGCCTCTC

CCTGTCTCTGGGTAAAGGTGGTGGTGGTTCTggaGGTggcGGTTCTGGTGGTGGTGG
TTCTCAAGATCGCCACATGATTAGAATGCGTCAACTTATAGATATTGTTGATCAGCT
GAAAAATTATGTGAATGACTTGGTCCCTGAATTTCTGCCAGCTCCAGAAGATGTAG
AGACAAACTGTGAGTGGTCAGCTTTTTCCTGTTTTCAGAAGGCCCAACTAAAGTC
AGCAAATACAGGAAACAATGAAAGGATAATCAATGTATCAATTAAAAAGCTGAAG
AGGAAACCACCTTCCACAAATGCAGGGAGAAGACAGAAACACAGACTAACATGC
CCTTCATGTGATTCTTATGAGAAAAAACCACCCAAAGAATTCCTAGAAAGATTCAA
ATCACTTCTCCAAAAGATGATTCATCAGCATCTGTCCTCTAGAACACACGGAAGTG
AAGATTCC (SEQ ID NO: 5).

It should be noted that, with respect to the nucleic acid molecules referred to in the present specification and claims, those skilled in the art will understand the actual inclusion of either, or both, complementary duplexes. For convenience, in this specification and claims, although in most cases only one strand is indicated, another strand complementary thereto is impliedly disclosed. In addition, the nucleic acid molecule sequences in the present disclosure include DNA forms or RNA forms, one of which is disclosed, meaning that the other is also disclosed.

In another aspect, the present disclosure provides an expression vector carrying the nucleic acid molecule as described above. The expression vector may include optional control sequences operably linked to the nucleic acid molecule. The control sequences are one or more control sequences that direct expression of the nucleic acid molecule in a host. The expression vector provided in the embodiments of the present disclosure can efficiently express the recombinant antibodies in suitable host cells, thereby effectively activating NK cells for treating or preventing diseases caused by tumor or virus infection.

In a yet another aspect, the present disclosure provides a recombinant cell carrying the nucleic acid molecule, expression vector, or recombinant antibody described above. The recombinant cell is obtained by transfecting or transforming the expression vector. According to some specific embodiments of the present disclosure, the recombinant cell can highly express the above recombinant antibody under suitable conditions, and the recombinant cell can be effectively used for treating or preventing diseases caused by tumor or virus infection.

It should be noted that "suitable conditions" in the description of the present disclosure refer to conditions suitable for the expression of the recombinant antibodies described in the present disclosure. Those skilled in the art can readily appreciate that suitable conditions for recombinant antibody expression include, but are not limited to, suitable transformation or transfection formats, suitable transformation or transfection conditions, healthy host cell status, suitable host cell density, suitable cell culture environment, and suitable cell culture time. The "suitable conditions" are not particularly limited, and one skilled in the art can optimize the conditions for the expression of the recombinant antibody optimally according to the specific circumstances of the laboratory.

In yet another aspect, the present disclosure provides a composition including the recombinant antibody, nucleic acid molecule, expression vector, or recombinant cell as described above. As described above, the recombinant antibody according to the embodiments of the present disclosure can effectively bind to CD16a or IL-21R protein molecules, so as to specifically recognize NK cells highly expressing the IL-21R and/or CD16a. Thus, the NK cells can be effectively activated, and the composition including the recombinant antibody also have significant effects in treating or preventing diseases caused by tumor or virus infection, having significant anti-tumor and antiviral capabilities. The composition is not particularly limited, and any one of the above-mentioned substances used in combination with any other substance shall fall within the scope of the composition described herein, such as a food composition or a pharmaceutical composition.

In one aspect, the present disclosure provides a medicament including the recombinant antibody nucleic acid molecule, expression vector, recombinant cell, or composition described above for use in treating or preventing tumor or virus infection. As described above, the recombinant antibody according to the embodiments of the present disclosure can effectively bind to CD16a and IL-21R proteins, so as to specifically recognize NK cells highly expressing the IL-21R and/or CD16a. the medicament prepared using the recombinant antibody and the corresponding series of substances also has significant effects in treating or preventing tumor or virus infection, having significant anti-tumor and antiviral capabilities.

According to some specific embodiments of the present disclosure, the tumor includes at least one of rectal cancer, lung cancer, breast cancer, liver cancer, gastric cancer, skin cancer, renal cancer, pancreatic cancer, and ovarian cancer.

According to some specific embodiments of the present disclosure, the virus infection includes at least one of HPV, HBV, influenza virus, and coronavirus.

A medicament provided according to some specific embodiments of the present disclosure includes a pharmaceutically acceptable carrier and an effective amount of the recombinant antibody active ingredient.

As used herein, the term "effective amount" or "effective dose" refers to an amount that produces an efficacy or activity and is acceptable to humans and/or animals.

As used herein, a "pharmaceutically acceptable" ingredient is one that is suitable for use in humans and/or mammals without causing undue adverse side effects (such as toxicity, irritation, and allergic response), i.e., one that has a reasonable benefit/risk ratio. The term "pharmaceutically acceptable carrier" refers to the carriers used in the administration of therapeutic agents, including various excipients and diluents.

The medicament of the present disclosure contains a safe and effective amount of the active ingredient according to the present disclosure and a pharmaceutically acceptable carrier. Such a carrier includes, but is not limited to: saline, buffer, glucose, water, glycerol, ethanol, and combinations thereof. Generally, the pharmaceutical preparations should be adapted to the administration mode, and the formulation of the medicament of the present disclosure can be injection, oral preparation (tablet, capsule, oral liquid), transdermal agent, and sustained-release agent. The preparations are prepared, for example, with conventional methods using physiological saline or aqueous solutions containing glucose and other adjuvants. The medicament is preferably produced under sterile conditions.

The effective amount of an active ingredient described herein may vary depending on the mode of administration and the severity of the disease to be treated. Selection of a preferred effective amount can be determined by one of ordinary skill in the art based on a variety of factors (e.g. through clinical trials). Such factors include, but are not limited to: pharmacokinetic parameters of the active ingredient such as bioavailability, metabolism, half-life, and the like; the severity of the disease to be treated by the patient, the weight of the patient, the immune status of the patient, the route of administration, etc. For example, several divided doses may be administered daily or the dose may be proportionally reduced as indicated by the exigencies of the therapeutic situation.

Pharmaceutically acceptable carriers described herein include, but are not limited to: water, saline, liposomes, lipids, proteins, protein-antibody conjugates, peptides, cellulose, nanogels, or combinations thereof. The selected carrier should be adapted to the administration manner, which is well known to those skilled in the art.

### Uses

In one aspect, the present disclosure provides the use of the above-mentioned recombinant antibody, nucleic acid molecule, expression vector, and recombinant cell in the culture and expansion of NK cells. The recombinant antibody, nucleic acid molecule, expression vector, and recombinant cell according to some specific embodiments of the present disclosure can be used to promote proliferation and expansion of the NK cells *in vitro,* and the expanded NK cells have better cytotoxicity.

According to some specific embodiments of the present disclosure, the NK cells are derived from peripheral blood or induced stem cells. NK cells are mainly distributed in peripheral blood. In addition, NK cells can also be obtained by inducing stem cells.

According to some specific embodiments of the present disclosure, NK cells are derived from human peripheral blood mononuclear cells, NK cells induced from human umbilical cord blood stem cells, NK cells induced from human embryonic stem cells, and NK cells induced from human iPSC. NK cells from peripheral blood mononuclear cells include lymphocytes and monocytes. According to some specific embodiments of the present disclosure, the above-mentioned recombinant antibody, nucleic acid molecule, expression vector, and recombinant cell can promote the *in vitro* proliferation of NK cells derived from the above sources with better expansion, and the expanded NK cells have higher cytotoxicity.

In a seventh aspect, the present disclosure provides the use of the above-mentioned recombinant antibody, nucleic acid molecule, expression vector, recombinant cell, or composition in the preparation of a medicament for treating or preventing tumor or virus infection. As described above, the recombinant antibody according to the embodiments of the present disclosure can effectively bind to CD16a and IL-21R proteins, so as to specifically recognize NK cells highly expressing the IL-21R and/or CD16a. The medicament prepared using the recombinant antibody and the corresponding series of substances can also have significant effects on the treatment or prevention of tumor or virus infection, having significant anti-tumor and antiviral capabilities.

According to some specific embodiments of the present disclosure, the above-mentioned uses may further include at least one of the following additional technical features.

According to some specific embodiments of the present disclosure, the tumor includes at least one of rectal cancer, lung cancer, breast cancer, liver cancer, gastric cancer, skin cancer, kidney cancer, pancreatic cancer, and ovarian cancer.

According to some specific embodiments of the present disclosure, the virus infection includes at least one of HPV, HBV, influenza virus, and coronavirus.

### Kit

In the last aspect, the present disclosure provides a kit including a recombinant antibody as described above. The recombinant antibody according to the embodiments of the present disclosure can efficiently bind to CD16a and IL-21R protein molecules, thereby specifically recognizing NK cells specifically expressing high levels of the IL-21R or CD16a. Thus, the recombinant antibodies can be used to prepare kits for detecting CD16a or IL-21R, which can be used in scientific studies, such as qualitative or quantitative detection of CD16a and/or IL-21R protein molecules in biological samples.

According to some specific embodiments of the present disclosure, the kit is used to detect CD16a and/or IL-21R.

### Disease Therapeutic Uses and Methods

In an aspect, the present disclosure provides the use of the above-mentioned recombinant antibody, nucleic acid molecule, expression vector, recombinant cell, composition, or medicament in the treatment or prevention of tumor or virus infection. As described above, the recombinant antibody can specifically recognize the above-mentioned CD16a and IL-21R proteins, and further can specifically recognize NK cells specifically highly expressing the IL-21R or CD16a. Thus, NK cells can be effectively activated, IFN-γ secretion can be enhanced, proliferation and NK cytotoxic activity can be enhanced, tumor cells can be effectively killed, NK cell maturation and NK cell receptor expression can be simultaneously accelerated, and exhaustion of NK cells in the tumor microenvironment can be effectively restored. Therefore, the recombinant antibody, the composition including the recombinant antibody, medicament according to the embodiments of the present disclosure, or a series of substances capable of expressing the recombinant antibody under appropriate conditions, for example, the above-mentioned nucleic acid molecule, expression vector, recombinant cell, etc., can effectively activate NK cells and can thus treat or prevent tumor or virus infection.

According to some specific embodiments of the present disclosure, the tumor includes at least one of rectal cancer, lung cancer, breast cancer, liver cancer, gastric cancer, skin cancer, kidney cancer, pancreatic cancer, and ovarian cancer. The virus infection includes at least one of HPV, HBV, influenza virus, and coronavirus.

In another aspect, the present disclosure provides a method for treating or preventing tumor or virus infection. According to some specific embodiments of the present disclosure, the method includes administering to the subject at least one of 1) the recombinant antibodies as described above; 2) the nucleic acid molecule as described above; 3) the expression vector as described above; 4) the recombinant cell as described above; 5) the composition as described above; or 6) the medicament, as described above. As described above, the recombinant antibody can specifically recognize the above-mentioned CD 16a and IL-21R proteins, and further can specifically recognize NK cells specifically highly expressing the IL-21R or CD16a. Thus, NK cells can be effectively activated, IFN-γ secretion can be enhanced, proliferation and NK cytotoxic activity can be enhanced, tumor cells can be effectively killed, NK cell maturation and NK cell receptor expression can be simultaneously accelerated, and exhaustion of NK cells in the tumor microenvironment can be effectively restored. Therefore, the above-mentioned recombinant antibody, composition including the recombinant antibody, medicament according to the embodiments of the present disclosure, or a series of substances capable of expressing the recombinant antibody under appropriate conditions, for example, the above-mentioned nucleic acid molecule, expression vector, recombinant cell, etc., can effectively activate NK cells and can thus treat or prevent tumor or virus infection.

According to some specific embodiments of the present disclosure, the tumor includes at least one of rectal cancer, lung cancer, breast cancer, liver cancer, gastric cancer, skin cancer, kidney cancer, pancreatic cancer, and ovarian cancer. The virus infection includes at least one of HPV, HBV, influenza virus, and coronavirus.

Hereinafter, embodiments will be described in detail. Where specific techniques or conditions are not specified in the examples, they are performed according to techniques or conditions described in the literature in the art or according to the product description. The reagents or instruments used are conventional products that can be obtained commercially without indicating the manufacturer.

### Example 1 Design and construction of bispecific antibody molecules anti-CD16a-FCy4-IL21

The specific experimental procedures of this example were as follows:

### 1.1 Acquisition of anti-CD 16a-FCγ4-IL21 fusion gene

### (1) Acquisition of Anti-CD16a-FCγ4 fusion gene

The Pgapza-CD16-FC4y4 plasmid was used as a template, and PCR was performed using primer 1 and primer 2 to obtain the anti-CD 16a-FCγ4 fusion gene, which had a nucleotide sequence as set forth in SEQ ID NO: 6. The PCR program was conducted as below: denaturation: 98°C 20 seconds; cycle: 98°C 10 seconds → 54°C 15 seconds → 72°C 40 seconds (30 cycles) extension: 72°C 2 minutes;

CAAGTTCAATTGGTTCAATCTGGTGCTGAGGTTAAGAAACCAGGTGAGT
CTTTGAAGGTTTCTTGTAAGGCTTCTGGTTATACTTTCACTTCTTACTATATGCATTG
GGTTAGACAAGCTCCAGGTCAAGGTTTGGAGTGGATGGGTATTATTAATCCATCTG
GTGGTTCTACTTCTTATGCTCAAAAGTTCCAAGGTAGAGTTACTATGACTAGAGATA
CTTCTACTTCTACTGTTTATATGGAGTTGTCTTCTTTGAGATCTGAAGATACTGCTGT
TTATTATTGTGCTAGAGGTTCTGCTTATTATTATGATTTCGCTGATTATTGGGGTCAA
GGTACTTTGGTTACTGTCTCTTCTGGTGGTGGTGGTTCTGGTGGTGGTGGTTCTGG
TGGTGGTGGTTCCTCTTACGTCTTGACTCAACCATCTTCTGTCTCTGTTGCTCCAGG
TCAAACTGCTACTATTTCTTGTGGTGGTCATAATATTGGTTCTAAGAATGTTCATTGG
TATCAACAAAGACCAGGTCAATCTCCAGTCTTGGTCATCTATCAAGATAACAAGAG
ACCATCTGGTATTCCAGAGAGATTCTCTGGTTCTAATTCTGGTAATACTGCTACTTT
GACTATTTCTGGTACTCAAGCTATGGATGAAGCTGATTACTATTGTCAAGTTTGGGA
TAATTATTCTGTCTTGTTCGGTGGTGGTACTAAGTTGACTGTTTTGGAGTCCAAATA
TGGTCCCCCATGCCCACCATGCCCAGCACCTGAGTTCGAAGGGGGACCATCAGTC
TTCCTGTTTCCCCCAAAACCCAAGGACACTCTCATGATCTCCCGGACCCCTGAGGT
CACGTGCGTGGTGGTGGACGTGAGCCAGGAAGACCCCGAGGTCCAGTTCAACTG

GTACGTGGATGGCGTGGAGGTGCATAATGCCAAGACAAAGCCGCGGGAGGAGCA
GTTCCAAAGCACGTACCGTGTGGTCAGCGTCCTCACCGTCCTGCACCAGGACTGG
CTGAACGGCAAGGAGTACAAGTGCAAGGTCTCCAACAAAGGCCTCCCGTCCTCCA
TCGAGAAAACCATCTCCAAAGCCAAAGGGCAGCCCCGAGAGCCACAGGTGTACA
CCCTGCCCCCATCCCAGGAGGAGATGACCAAGAACCAGGTCAGCCTGacCTGCCTG
GTCAAAGGCTTCTACCCCAGCGACATCGCCGTGGAGTGGGAGAGCAATGGGCAGC
CGGAGAACAACTACAAGACCACGCCTCCCGTGCTGGACTCCGACGGCTCCTTCTT
CCTCTACAGCAGACTAACCGTGGACAAGAGCAGGTGGCAGGAGGGGAATGTCTTC
TCATGCTCCGTGATGCATGAGGCTCTGCACAACCACTACACACAGAAGAGCCTCTC
CCTGTCTCTGGGTAAA (SEQ ID NO:6).

### (2) Acquisition of GS-II,21 fusion gene

The Ppicza-rdna-FC-Y407T-IL21 plasmid was used as a template, PCR was performed using primer 3 and primer 4 to obtain the GS-II,21 fusion gene, which had a nucleotide sequence as set forth in SEQ ID NO: 7. The PCR program was conducted as follows: denaturation: 98 20 seconds; cycle: 98°C 10 seconds → 54°C 15 seconds → 72°C 30 seconds (30 cycles) extension: 72°C 5 minutes.

GGTGGTGGTGGTTCTggaGGTggcGGTTCTGGTGGTGGTTCTCAAGAT
CGCCACATGATTAGAATGCGTCAACTTATAGATATTGTTGATCAGCTGAAAAATTAT
GTGAATGACTTGGTCCCTGAATTTCTGCCAGCTCCAGAAGATGTAGAGACAAACT
GTGAGTGGTCAGCTTTTTCCTGTTTTCAGAAGGCCCAACTAAAGTCAGCAAATACA
GGAAACAATGAAAGGATAATCAATGTATCAATTAAAAAGCTGAAGAGGAAACCAC
CTTCCACAAATGCAGGGAGAAGACAGAAACACAGACTAACATGCCCTTCATGTGA
TTCTTATGAGAAAAAACCACCCAAAGAATTCCTAGAAAGATTCAAATCACTTCTCC
AAAAGATGATTCATCAGCATCTGTCCTCTAGAACACACGGAAGTGAAGATTCCTAG
(SEQ ID NO:7).

The sequences of the primers 1-4 are shown in Table 1:

**Table 1:**

| No. | Name | Sequence |
|---|---|---|
| Primer 1 | Fw-CD16-XhoI | CTCTCGAGAAAAGACAAGTTCAATTGGTTC(SEQ ID NO:8) |
| Primer 2 | RV-T407Y | CGGTTAGTCTGCTGTAGAGGAAGAAG(SEQ ID NO:9) |
| Primer 3 | FW-T407Y | CTTCTTCCTCTACAGCAGACTAACCG(SEQ ID NO:10) |
| Primer 4 | RV-NOTI | TTCGCGGCCGCCTAGGAATCTTCACTTCCG(SEQ ID NO:11) |

Agarose gel electrophoresis was performed on the expanded products of the above-mentioned two steps, and according to the operation steps of the DNA gel recovery kit, the bands corresponding to the two target genes were cut and collected to obtain the fusion gene of Anti-CD16a-FCγ4 and GS-IL21, namely, obtaining a first target gene fragment and a second target gene fragment;

### (3) Acquisition of Anti-CD16a-FCγ4-IL21 fusion gene

The Anti-CD16a-FCγ4 and GS-IL21 fusion genes obtained in the above-mentioned steps were used as a template, PCR was performed by using primer 1 and primer 4 to obtain the Anti-CD16a-FCγ4-IL21 fusion gene, and the PCR program was: denaturation: 98°C 20 seconds; 3 Cycle: 98°C 10 seconds- 54°C 20 seconds- 72°C 40 seconds (3 cycles); extension: 72°C 2 minutes; primers were then added: denaturation: 98°C 20 seconds; 3 cycle: 98°C 10 seconds→ 54°C 20 seconds→ 72°C 40 seconds (30 cycles); extension: 72°C 10 minutes. Agarose gel electrophoresis was performed on the expanded product, and then a DNA gel recovery kit was used to recover a fragment of gene, which had Xho I and Not I enzyme cleavage sites at two ends, named as Anti-CD16a-FCγ4-IL21.

The above-mentioned Pgapza-CD16-FC4γ4 and Ppicza-rdna-FC-Y407T-IL21 plasmids were constructed by the Applicant according to conventional expression vector construction methods.

### 1.2 Construction of Pichia pastoris expression vector of anti-CD16a-FCγ4-IL21 fused protein

The gene sequence encoding the anti-CD16a-FCγ4-IL21 fused protein obtained in step 1.1 (SEQ ID NO: 5) and *Pichia pastoris* expression plasmid PGAPzα-rDNA-NTS stored in the laboratory were both double digested with restriction endonucleases, i.e., Xhol and Not I. The digested anti-CD16a-FCγ4-IL21 fused protein gene sequence product gene was recovered using a PCR product recovery kit (Axygen), and the digested plasmid was recovered using a DNA gel recovery kit (Axygen). The operation procedures can refer to the kit instructions. The recovered digested fused protein gene was linked with the digested vector using T4 DNA ligase, and the constructed plasmid structure image is illustrated in FIG. 1. The enzyme-linked product was transformed into *E. coli* TOP10 competent cells. The conversion procedure was as below: 10 µl of the enzyme-linked product was added into 100 µL of *E. coli* competent cells, mixed well, allowed to stand on ice for 15 to 30 min, and shaken well every 5 min. Then, the mixture was subjected to heat shock at 42°C for 90 seconds, standing on ice for 3 to 5 min, 400 µl of LB liquid culture medium without resistance was added, the mixture was subjected to recovery culturing at 37°C on a shaker for 45 min, and plated on Zeocin resistant plates. After the clonal colonies grew on the plate, a single colony was selected and cloned into a shake tube containing LZ liquid culture medium (LB culture medium with Zeocin resistance), and cultured at 37°C for 6 to 8 hours. Then, the bacterial solution is used as a template for PCR identification, and the obtained positive clone is named as PGAP-zα-anti-CD16a-FCγ4-IL21. The bacterial solution corresponding to the positive clone was extracted with the plasmid small-scale extraction kit and sent to General Biological for sequencing. After sequencing, the sequencing results were consistent with the theoretical expected result, and the clone was constructed correctly.

### Example 2 Expression and purification of the bispecific antibody molecule anti-CD16a-FCy4-IL21

### 2.1 Expression of anti-CD16a-FCγ4-IL21 bispecific antibody molecule

The positive clone constructed in Example 1 was expanded and cultured using 50 mL of LZ liquid culture medium, and a medium amount of plasmid PGAP-zα-anti-CD16a-FCγ4-IL21 was extracted using a kit (Axygen Company). The expression plasmid PGAP-zα-anti-CD16a-FCγ4-IL21 prepared in the previous step was linearized with endonuclease Spe I and recovered by ethanol precipitation. Ethanol precipitation steps: 1) two times the volume of anhydrous ethanol and 0.1 times the volume of 3M sodium acetate were added to the enzyme digestion reaction system and precipitated at -20°C for more than 2 hours; 2) the mixture was centrifuged at 12,000 g for 10 min and the supernatant was discarded; 3) the precipitate was resuspended in 300 µL of 70% ethanol, centrifuged at 12,000 g for 10 min and the supernatant was discarded; and 4) the mixture was dried at 37°C, and re-suspended with deionized water, the concentration was determined and adjusted to 0.5 to 1.0 µg/µL.

Preparation of yeast competent: preparing steps: 1) the well-frozen *Pichia pastoris* X33 strain was streaked on a YPD plate and cultured at 30°C in a yeast incubator; 2) after the colonies grew to about 1 mm in diameter, they were picked and cultured in liquid 4 mL of YPD culture medium on a yeast shaker at 30°C; 3) cultured for 24 to 48 hours, 1 mL of the broth was inoculated into 50 mL of fresh YPD culture medium; 4) when the OD600 value reached 1 to 1.5, the bacterial solution was transferred to a 50 mL centrifuge tube, centrifuged at 1,500 g for 5 min at 4°C, and the supernatant was discarded; 5) resuspended in 50 mL of ice water, and centrifuged at 1,500g for 5 min at 4°C, the supernatant was discarded and repeated once; 6) resuspended in 50 mL of 1 M cold sorbitol, centrifuged at 1,500 g for 5 min at 4°C, the supernatant was discarded and repeated once; and 7) resuspended in 500 µL of 1 M cold sorbitol.

Electroporation steps: 100 µL of the above competent cells was taken in a sterile precooled electroporation cuvette, 10 µL of linearized plasmid (5 to 10 µg) was added, and mixed well. The parameters of the electroporator were set as 2000 V, 200 Ω, 25 µF. Electroporation was performed, and after electroporation, 1 mL of 1 M cold sorbitol was immediately added into the electroporation cuvette. The mixture was allowed to stand on ice for 5 to 10 min, then the whole bacterial solution was transferred into a 50 mL centrifuge tube filled with 1 mL of YPD, to recover at 30°C and 225 rpm for 2 hours. At the end of recovery, 100 to 1000 µL of the above bacterial solution was coated onto a YPDZ (plate containing Zeocin resistance) plate, and the plate was placed in a constant temperature yeast incubator at 30°C for culture.

Positive expression clone selection: after the yeast colonies grew out from the above-mentioned YPDZ plate, several colonies were picked and cultured in 2 mL of BMGY culture medium at a constant temperature of 28°C on a yeast shaker until the bacterial solution became milky. 1 mL of bacteria solution was frozen and preserved, after centrifuging at 12,000 g for 5 min, the supernatant of the remaining 1 mL of solution was taken for identification and primary screening using Dot blot with Anti-human FCγ4 antibody. The yeast strains with relatively high expression were picked out, and then the yeast strains with relatively high expression were coated on the YPD plate containing zeocin. After the clones were grown, several clones were selected to shake the bacteria in the above way and the supernatant of the bacteria was obtained, and the Western Blot identification and analysis was carried out with Anti-human FCγ antibody. The strain with high expression (34-3) was screened, and the remaining 1 mL solution of the corresponding strain was frozen and preserved; the high expression strain was a *Pichia pastoris* strain capable of expressing anti-CD16a-FCγ4-IL21 complex protein.

Specific experimental results were shown in FIG. 3, a yeast expression strain of the fused protein was obtained by YPDZ resistance culture plate selection. Using Dot blot for preliminary screening, some clones with relatively high expression levels can be obtained preliminarily (such as No. 66, 91, 98, 34 in FIG. 3B), followed by further confirmation of the comparison by Western Blot, and yeast expression clones with higher expression levels can be selected for subsequent protein expression purification.

### 2.2 Fermentation, purification, and identification of anti-CD16a-FCγ4-IL21 bispecific antibody molecule

### (1) Fermentation

Culturing expression strain: fermentation of the recombinant strains was carried out according to the fermentation instruction manual of Invitrogen. The screened high-expression strains were inoculated into a shake tube containing 4 mL of YPD culture medium, and the mixture was cultured at 30°C for 24 to 48 hours on a shaker until the OD600 was 2 to 6, i.e., the primary fermentation seed solution. 1 mL of the primary fermentation seed solution was transferred to an Erlenmeyer flask containing 200 mL of BMGY culture medium, and the mixture was cultured at 30°C for 12 to 24 hours on a shaker to obtain a secondary fermentation seed solution. 200 mL of the secondary seed solution was totally inoculated into a fermenter containing 6 L of BMGY culture medium. Setting parameters: culture temperature was 30°C, pH was 7.0, dissolved oxygen and rotation speed were monitored, and fermentation culture was started. BMGY culture medium was used in the growth stage of bacteria. When the dissolved oxygen rose rapidly, the basal glycerol was consumed in the culture medium. Glycerol feeding was started. The glycerol feeding rate was as follows: 90 mL/h, the fermentation temperature was adjusted to 25°C, and continued until the end of fermentation.

Clarifying culture solution: after completion of the fermentation, the mixture was centrifuged at 10,000 g for 30 min, and the supernatant was collected and clarified by filtration through 0.22 um and 500 kDa microfiltration membranes, after which the pH was adjusted to 7.4.

The parameters of the fermentation process were monitored as shown in FIG. 4. The whole fermentation process lasted for 50 hours, and the fermentation process was stable and easy to repeat. As can be seen from FIG. 4B, glycerol feeding restriction culture was performed from the end of the basal glycerol culture phase to induce protein expression (20 hours), and protein accumulation continued throughout the process, with little difference in protein accumulation between 43-50 h, and the end of fermentation was determined.

### (2) Purification

Protein capturing: the recombinant fused protein was captured using an affinity chromatography column, a MabSelect column. The specific steps were: 1) the column was flushed with deionized water in 5 column volumes; 2) the column was flushed with PBS in three column volumes; 3) loading; 4) subsequent to the loading, the column was flushed with PBS in three column volumes; 5) washing impurity: the column was flushed with citrate buffer having pH 5.5 in 5 column volumes; and 6) the target protein was eluted with pH 3.0 citrate buffer, and the collected eluate was concentrated for subsequent purification.

Fine purifying: the Superdex200 molecular sieve was equilibrated with PBS buffer using AKTA PURE 25, and after equilibration, the neutral eluate was loaded, followed by washing with PBS buffer, and the eluted sample was collected to obtain anti-CD 16a-FCγ4-IL21 recombinant protein.

By centrifugation and two-step purification, the protein purity was more than 95% as identified by SDS-PAGE, HPLC (FIG. 5A, C).

### (3) Identification

Identifying recombinant proteins: the purified protein was identified by SDS-PAGE, HPLC, and dynamic light scattering. Specific experimental results are shown in FIG. 4B, in which, the molecular weight of anti-CD16a-FCγ4-IL21 protein is about 135 kDa, which is consistent with the expectation. By identifying with dynamic light scattering, the anti-CD16a-FCγ4-IL21 was about 10 nm in diameter and had good intermolecular homogeneity.

### Example 3 Verification of NK cell activation function induced in vitro by bispecific antibody anti-CD16a-FCγ4-IL21

Human PBMCs were isolated by Ficoll density gradient centrifugation. The isolated PBMCs were diluted with RPMI 1640 containing 10% FBS to a density of 1.0 × 10⁶/mL. 10 mL of diluted cells was added to the T25 culture flask. After 12 hours of culture, anti-CD16a-FCγ4-IL21 fused protein was added to the culture flask at a final concentration of 20 nM. After 24 hours of culture, the phenotype changes of NK cells were detected by flow cytometer, mainly including the expression of major activating molecules CD69, NKp44, and killing molecules 4-1BB, TRAIL, and Granzyme B on the surface of NK cells.

Specific experimental results were shown in FIG. 6, the expression levels of major activating molecules CD69, NKp44, and killing molecules 4-1BB, TRAIL, and Granzyme B on the surface of NK cells were significantly up-regulated in the experimental group supplemented with anti-CD 16a-FCγ4-IL21 fused protein, compared with the control group PBS (Control group), and thus it was determined that NK cells were efficiently activated by anti-CD16a-FCγ4-IL21 fused protein.

### Example 4 NK cell expansion induced in vitro by bispecific antibody molecule anti-CD16a-FCγ4-IL21 and evaluation of the expansion effect

### 4.1 Purity and quantity detection ofNK cells expanded by anti-CD16a-FCγ4-IL21

The fused protein was used to coat the T75 culture flask 24 h in advance, and 7.5 mL of coating solution was taken from each flask, to cover the bottom of the flask body. Coating overnight in a refrigerator at 4°C.

After PBMCs were isolated from the blood samples, they were counted (Mindray counter) and the percentage of each cell subset in PBMCs was recorded. Inoculating at a density of 1.5*10⁶/mL (20 mL culture volume). The total number of cells was 30*10⁶. This was Day 0. Meanwhile, the KBM581 culture medium without anti-CD16a-FCγ4-IL21 was supplemented with 1% IL2 (1000 IU/mL) and 5% of the corresponding PBMC autologous plasma.

On Day 3, the IL2 factor at a volume of 1% of the corresponding culture volume was supplemented.

On Day 5, the color of the culture medium and cell adherence state at the bottom of the T75 flask body were observed, 5 to 10 mL of KBM581 culture medium containing anti-CD16a-FCγ4-IL21 was supplemented as needed, and IL2 (1000 IU/mL) factor at a volume of 1% of the supplemented solution was added.

On Day 6, samples were taken for counting for cell passage and expanded culture (1.3*10⁶/mL). After staining and labeling (CD16a/CD45/CD56), the percentage of NK cell subset and the percentage of CD56+ cell subset were detected by flow cytometer, and the total cell number was calculated and the NK cell number was calculated according to the results of flow purity detection. KBM581 culture medium containing anti-CD16a-FCγ4-IL21 was used for cell passage and expanded culture from Day 5, which was supplemented with 1% IL2 and 2% autologous plasma.

On Day 7, the color of the culture medium and the adherent growth of the cells on the flask body were observed, and fluid supplementation was performed as needed (with the addition of IL2 factor at a volume of 1% of fluid supplementation).

On Day 8, samples were counted. Cell passage and expansion were performed as needed (T75 or T175 flasks, 1.4*10⁶/mL) and supplemented with 1% IL2 factor, and 2% autologous plasma in culture medium.

On Day 9, the color of the culture medium and the adherent growth of the cells on the flask body were observed, and fluid supplementation was performed as needed (with the addition of IL2 factor at a volume of 1% of fluid supplementation).

On Day 10, samples were taken for counting and cell passage and expanded culture (1.5*10⁶/mL). After staining and labeling (CD16a/CD45/CD56), the percentage of NK cell subset and the percentage of CD56+ cell subset were detected by flow cytometer, and the total cell number was calculated and the NK cell number was calculated according to the results of flow purity detection.

On Day 12, the color of the culture medium and the adherent growth of the cells on the flask body were observed, and fluid supplementation was performed as needed (with the addition of IL2 factor at a volume of 1% of fluid supplementation).

On Day 13, samples were taken for counting and cell passage and expanded culture (1.5*10⁶/mL). After staining and labeling (CD16a/CD45/CD56), the percentage of NK cell subset and the percentage of CD56+ cell subset were detected by flow cytometer, and the total cell number was calculated and the NK cell number was calculated according to the results of flow purity detection.

On Day 15, samples were counted. Cell passage and expansion were performed as needed (T175 flasks or culture bags, 1.5*10^6/mL) and supplemented with 1% IL2 factor in culture medium.

On Day 17, samples were taken for counting, after staining and labeling (CD16a/CD45/CD56), the percentage of NK cell subset and the percentage of CD56+ cell subset were detected by flow cytometer, and the total cell number was calculated and the NK cell number was calculated according to the results of flow purity detection. NK cell proportion and number were plotted as a function of time by NK purity and cell number at Day 0, 6, 10, 13, 17.

Specific experimental results were shown in A to C of FIG. 7, the expansion of NK cells derived from PBMCs by using anti-CD16a-FCγ4-IL21 can effectively expand NK cells and improve the purity and expansion factor of NK cells, and the highest proportion of NK cells in the whole culture process can reach about 65%.

### 4.2 Analysis of the subset of PBMC-derived NK cells expanded by bispecific antibody molecules

(1) Inducing intracellular molecular labeled cells with monensin: 4 × 10⁶ cultured cells (2 mL) were taken and placed in a 6-well plate, 10 µL of monensin (0.5 µg/µL) was added, cells were incubated at 37°C, 5% CO₂ constant temperature incubator for induction for 4 hours;'.
(2) Preparing single cell suspension: 9.0 × 10⁶ cultured cells from the surface molecular labeled cells were taken into a 15 mL centrifuge tube, centrifuged at 400 g for 8 min to collect the cells, washed twice with 10 mL of 1X PBS, and finally resuspended into a single cell suspension with 0.81 mL of 1X PBS. After induction of intracellular molecular labeled cells with monensin, the cells were washed twice with 5 mL of 1X PBS and finally resuspended with 180 µL of 1X PBS into a single cell suspension.
(3) Blocking: 90 µL of mouse serum was added to the surface molecular labeled cells, mixing well, 20 µL of mouse serum was added to the intracellular molecular labeled cells, mixing well, and allowing to stand at room temperature for 15 to 30 min.
(4) Labeling antibodies: after blocking, the surface molecular labeled cells were divided into 9 flow tubes at 0.09 mL/tube. If multiple batches of cells were detected at the same time, the cells in tubes 1 to 8 can be labeled after mixing several batches of cells in equal amounts, and the number of cells in each tube was 0.8 to 1 × 10⁶ cells. Sample tube 1 was a surface molecular labeled, and the number of cells was 0.8 to 1 × 10⁶. Sample tube 2 was an intracellular molecular marker, and the number of cells was 3 to 4 × 10⁶, which were added with the corresponding fluorescently labeled antibody, mixed well and the mixture was allowed to stand for 30 min at 4°C, protected from light (mixed well every 15 min during the process). Specific experimental groups are as shown in Table 2.

**Table 2: Sample tube for cell surface molecule detection and experimental operation table**

| No. | Groups | Fluorescent labeled antibody | Amount |
|---|---|---|---|
| 1 | Blank control tube | / | / |
| 2 | | mouse IgG1- Alexa Flour 647 | 0.5 µL |
| | | mouseIgG1-percp cy5.5 | 0.5 µL |
| | | mouse IgG1-BV785 | 0.5 µL |
| 3 | Single-labeled control tube | CD16a- Percpcy5.5 | 0.5 µL |
| 4 | | CD56- BV785 | 0.5 µL |
| 5 | | DAPI | 5 µL |
| 6 | | BVS10-CD19 | 1 µL |
| 8 | | BV650-CD14 | 1 µL |
| Sample tube | | CD16a-Percp cy5.5 | 0.5 µL |
| | | CD56- BV785 | 0.5 µL |
| | | CD14-BV650 | 1 µL |
| | | CD19-BV510 | 1 µL |

(5) Washing: 1 mL of 1X PBS was added to each tube of surface molecular labeled cells, and the cells were harvested by centrifuging at 4°C, 400 g for 8 min. The cells were washed 2 times with 1 mL of 1X PBS. Finally, 200 µL of PBS was added to each tube to resuspend the labeled cells, 5 µL of DAPI (50 µg/mL, 40x) was added to the experimental group, and the tube was transferred to the machine for detection.
(6) Testing: the flow cytometer was calibrated and adjusted according to the instructions for use. The blank control tube samples were used to adjust the forward and side scatter voltages. The single-labeled control samples were used to adjust the fluorescence compensation of each channel. After the cell gates were delineated during the assay, 1 × 10⁴ cells were collected in each sample gate.

Specific experimental results are shown in FIG. 7-D, cell subset analysis of the expanded cells showed that in the PBMCs cultured and expanded with anti-CD 16a-FCγ4-IL21, the proportion of NK cells was up to 61%, the proportion of NKT was about 10%, and the proportion of total CD56 positive cells was about 71%, and the remaining cell subsets were relatively low in the expanded PBMCs.

### 4.3 Detection of killing activity of NK cells expanded by bispecific antibody molecules

### (1) Preparation of target cell K562 suspension

Cell counting: the cells were suspended in 1640 culture medium containing 0.5% FBS (the culture medium used for target cells), counted and the cell density was adjusted to 1 × 10⁶/mL.

CFSE staining: CFSE (working concentration: 5 µM) was added to the cell suspension, and the cells were pipetted and mixed well with 1 mL pipette, followed by vortex, incubating for 15 min in a 37°C incubator protected from light, and taking out every 5min to vortex well.

Stop staining: the staining was stopped by adding 5 volumes of complete culture medium precooled at 4°C (the culture medium used for target cells) and incubated on ice for 5 min, followed by centrifuging at 140 g and 4°C for 5 min.

Washing cells: complete culture medium precooled at 4°C (the culture medium used for target cells) was used to re-suspend the cells, and the cells were centrifuged at 140 g and 4°C for 5 min. The washing was repeated twice.

Counting: the cells were resuspended and counted, and the cell density was adjusted to 2 × 10⁵/mL.

Plating: 100 µl of target cell suspension was added into each well of the 96-well round bottom plate so that the final cell number in each well is 20,000 cells/well.

### (2) Preparation and Addition of NK Cells

NK cells were prepared at a suitable density.
The E-Plate 16 was taken and placed on a super-clean bench;
100 µl of NK cell suspension at different effective target ratios (CD56+: K562) (1:1, 2:1, 4:1, and 8:1) was added to the culture plate. In addition, three groups of controls were provided, i.e., target cells stained with CFSE only (natural mortality of target cells was detected); only effector cells (demonstrating that effector cells do not contain CFSE non-specific staining); and target cells + Tween-20 (as positive control for apoptosis of target cells);

Co-incubation, NK cells were added to each well in a pre-designed order. The target cells were contacted thoroughly by centrifugation at 120 g and at room temperature for 2 min. The cells were returned for incubating in a 37°C incubator for 4 hours.

At the end of incubation, 5 µl of PI was added, mixed well, incubated for 5 min protected from light, and detected on the machine.

Analysis of results: percentage of killing = [(death rate (%) of target cells in experimental group-death rate (%) of target cells)/(100%-natural death rate (%) of target cells)] x 100%).

Specific experimental results are shown in FIG. 7-E, the cytotoxicity of the expanded cells was evaluated, and the expanded cells can effectively kill K562 cells.

In conclusion, the experimental results of the example demonstrate that anti-CD 16a-FCγ4-IL21 have the activity of activating and expanding NK cells *in vitro,* and the expanded NK cells have better cytotoxicity.

In the specification, references to descriptions of the terms "one embodiment", "some embodiments", "examples", "specific examples", or "some examples", etc., mean that a particular feature, structure, material, or characteristic described in connection with the embodiment or example is included in at least one embodiment or example of the present disclosure. In the specification, schematic representations of the above terms are not necessarily directed to the same embodiment or example. Furthermore, the particular features, structures, materials, or characteristics described may be combined in any suitable manner in any one or more embodiments or examples. Furthermore, combinations and combinations of the various embodiments or examples and features of the various embodiments or examples described in this specification can be made by those skilled in the art without departing from the scope of the present disclosure.

While embodiments of the present disclosure are illustrated and described above, it will be understood that the above-described embodiments are illustrative and not restrictive. Those skilled in the art can make changes, modifications, substitutions, and alterations to these embodiments without departing from the scope of the present disclosure.

## Claims

1. A recombinant antibody, comprising:
an IL-21 molecule; and
a CD16a antibody, the CD16a antibody comprising a CD16a single-chain antibody and an Fc region, an N-terminus of the Fc region being linked to a C-terminus of the single-chain antibody, the CD16a single-chain antibody comprising a heavy chain variable region and a light chain variable region, and a C-terminus of the Fc region being linked to am N-terminus of the IL21 molecule.

2. The recombinant antibody according to claim 1, further comprising a connecting peptide 1, wherein:
an N-terminus of the connecting peptide 1 is linked to a C-terminus of the heavy chain variable region of the CD16a single-chain antibody, and a C-terminus of the connecting peptide 1 is linked to an N-terminus of the light chain variable region of the CD16a single-chain antibody; and
optionally, the connecting peptide 1 has an amino acid sequence as set forth in SEQ ID NO: 1.

3. The recombinant antibody according to claim 1, further comprising a connecting peptide 2, wherein:
an N-terminus of the connecting peptide 2 is linked to the C-terminus of the IL-21 molecule, and a C-terminus of the connecting peptide 2 is linked to the N-terminus of the Fc region; and
optionally, the connecting peptide 2 has an amino acid sequence as set forth in SEQ ID NO: 2.

4. The recombinant antibody according to claim 3, wherein at least a portion of the Fc region is derived from at least one of a murine antibody, a human antibody, a primate antibody, or a mutant thereof.

5. The recombinant antibody according to claim 3 or 4, wherein at least a portion of the Fc region is derived from human IgG or a mutant thereof.

6. The recombinant antibody according to any one of claims 3 to 5, wherein at least a portion of the Fc region is derived from human Fcγ4 or a mutant thereof.

7. The recombinant antibody according to any one of claims 3 to 6, wherein the Fc region has an amino acid sequence as set forth in SEQ ID NO: 3.

8. The recombinant antibody according to any one of claims 1 to 7, having an amino acid sequence as set forth in SEQ ID NO: 4.

9. A nucleic acid molecule, encoding the recombinant antibody according to any one of claims 1 to 8.

10. The nucleic acid molecule according to claim 9, having a nucleotide sequence as set forth in SEQ ID NO: 5.

11. An expression vector, carrying the nucleic acid molecule according to claim 9 or 10.

12. A recombinant cell, carrying the nucleic acid molecule according to claim 9 or 10, the expression vector according to claim 11, or the recombinant antibody according to any one of claims 1 to 8.

13. A composition, comprising the recombinant antibody according to any one of claims 1 to 8, the nucleic acid molecule according to claim 9 or 10, the expression vector according to claim 11, or the recombinant cell according to claim 12.

14. Use of the recombinant antibody according to any one of claims 1 to 8, the nucleic acid molecule according to claim 9 or 10, the expression vector according to claim 11, the recombinant cell according to claim 12, or the composition according to claim 13 in the preparation of a medicament for treating or preventing tumor or virus infection.

15. The use according to claim 14, wherein: the tumor comprises at least one of rectal cancer, lung cancer, breast cancer, liver cancer, gastric cancer, skin cancer, kidney cancer, pancreatic cancer, and ovarian cancer; and
the virus infection comprises at least one of HPV, HBV, influenza virus, and coronavirus.

16. Use of the recombinant antibody according to any one of claims 1 to 8, the nucleic acid molecule according to claim 9 or 10, the expression vector according to claim 11, and the recombinant cell according to claim 12 in the culture and expansion of NK cells.

17. The use according to claim 16, wherein the NK cells are derived from peripheral blood or induced from stem cells.

18. The use according to claim 16, wherein the NK cells comprise at least one of NK cells derived from human peripheral blood mononuclear cells, NK cells induced from human umbilical cord blood stem cells, NK cells induced from human embryonic stem cells, and NK cells induced from human induced pluripotent stem cells (iPSCs).

19. A medicament, comprising the recombinant antibody according to any one of claims 1 to 8, the nucleic acid molecule according to claim 9 or 10, the expression vector according to claim 11, the recombinant cell according to claim 12, or the composition according to claim 13, wherein the medicament is used for treating or preventing tumor or virus infection.

20. The medicament according to claim 19, wherein:
the tumor comprises at least one of rectal cancer, lung cancer, breast cancer, liver cancer, gastric cancer, skin cancer, kidney cancer, pancreatic cancer, and ovarian cancer; and
the virus infection comprises at least one of HPV, HBV, influenza virus, and coronavirus.

21. A kit, comprising the recombinant antibody according to any one of claims 1 to 8.

22. The kit according to claim 21, wherein the kit is used for detecting CD16a and/or IL-21R.

23. Use of the recombinant antibody according to any one of claims 1 to 8, the nucleic acid molecule according to claim 9 or 10, the expression vector according to claim 11, the recombinant cell according to claim 12, the composition according to claim 13, or the medicament according to any one of claims 19 and 20 in the treatment or prevention of tumor or virus infection.

24. The use according to claim 23, wherein:
the tumor comprises at least one of rectal cancer, lung cancer, breast cancer, liver cancer, gastric cancer, skin cancer, kidney cancer, pancreatic cancer, and ovarian cancer; and
the virus infection comprises at least one of HPV, HBV, influenza virus, and coronavirus.

25. A method for treating or preventing tumor or virus infection, comprising administering to a subject at least one of the following:
1) the recombinant antibody according to any one of claims 1 to 8;
2) the nucleic acid molecule according to claim 9 or 10;
3) the expression vector according to claim 11;
4) the recombinant cell according to claim 12;
5) the composition according to claim 13; or
6) the medicament according to any one of claims 19 and 20.

26. The method according to claim 25, wherein the tumor comprises at least one of rectal cancer, lung cancer, breast cancer, liver cancer, gastric cancer, skin cancer, kidney cancer, pancreatic cancer, and ovarian cancer; and
the virus infection comprises at least one of HPV, HBV, influenza virus, and coronavirus.
